# EUROPEAN PATENT APPLICATION

(11) **EP 1 941 894 A1**
(43) Date of publication of application: **09.07.2008**
(21) Application number: 06757778.3
(22) Date of filing: 06.06.2006
(51) Int. Cl.: A61K 36/48, A61K 33/00, A61P 15/12

(54) **ISOFLAVONE COMPOSITION FOR THE TREATMENT OF MENOPAUSAL PHYSIOLOGICAL DISORDERS AND SYMPTOMS**

(30) Priority: 12.08.2005 MX PA05008573
(71) Applicant: WORLD-TRADE IMPORT-EXPORT, WTIE, A.G., 6302 Zug (CH)
(72) Inventor: ESPINOSA ABDALA, Leopold de Jesùs, Col. Colinas de San Javier C.P. 45110 Zapopan, Jalisco (MX); GARCÍA ARMENTA, María Elena, C.P. 45020 Guadalajara, Jalisco (MX); SANTOS MURILLO, Josefina, C.P. 44600 Zapopan, Jalisco (MX); ÁLVAREZ OCHOA, Victor Guillermo, C.P. 45222 Zapopan, Jalisco (MX)
(86) International application number: PCT/MX2006/000045
(87) International publication number: WO 2007/021166

(57) **Abstract**

The invention relates to phytochemical compounds in general having biological properties that have an effect on a variety of biochemical and physiological processes in the human organism. In particular, the invention relates to phytoestrogens having properties that are potentially beneficial for the health and, more specifically, to an isoflavone composition for the treatment of menopausal symptoms. The invention is **characterized in that** the composition comprises: *Glycine max* isoflavones; at least one or a mixture of vitamins which are selected from the group containing vitamin A, niacin, vitamin. B, vitamin C; vitamin D, vitamin E, folic acid and/or vitamin K, pantothenic acid and at least one or a mixture of minerals which are selected from the group containing chromium, magnesium, manganese, calcium, copper, zinc, iron, potassium, phosphorus or any other mineral suitable for human cosumption.

## Description

### FIELD OF THE INVENTION

The present invention is related generally to phytochemical compounds having biological properties affecting a variety of biochemical and physiological processes of human body, more particularly is related to phytoestrogens having properties potentially beneficial for health, and specifically it refers to an isoflavone composition for the treatment of menopausal symptoms.

### BACKGROUND OF THE INVENTION

Menopause is characterized by the disappearance of ovulation and therefore of menstruation in women. Around age 40 to 45 the ovulation cycles begin to diminish and this is the start of climacterium or transition period between the beginning of a decrease in the releasing of sexual hormone (estrogens and progesterone) by the ovaries and the end of functions thereof. Normally, this occurs at ages between 42 and 55, but it can occur prematurely as a consequence of radiotherapy, surgery or ovarian disorders.

Therefore climacterium occurs from 2 to 4 years before menopause

Hormonal fluctuations give rise to a great variety of clinical manifestations that occur with greater or lesser intensity in most women constituting the menopausal syndrome; these manifestations can be divided into:

Menstrual changes:- in the pre-menopausal period, the cycles became irregular and anovulatory.

Vasomotor syndromes.- the most common symptom are hot flashes, which occur suddenly, preferably in warm environments and during night. Hot flashes are secondary to local dilatation of blood vessels and they are manifested as skin reddening, heavy perspiration, sensation of heat in thorax and head, together with discomfort; palpitations are also frequent:

Urogenital system manifestations. A diminution in the vaginal secretions occurs, and this causes itching, burning and dyspareunia (pain during intercourse).

Osteoporosis. It is a consequence of increased bone re-absorption linked to a declination in bone formation. Osteoporosis is progressive, weakens bone resistance, thereby producing fractures with greater ease, preferably of wrist and femoral neck.

Cardiovascular manifestations. A greater increase of cholesterol occurs and a tendency to increase blood pressure.

Emotional disorders. They essentially consist in anxiety, nervousness, irritability and moderated depressions.

There are many symptoms and negative physiological effects caused by menopause, for which exist several control methods and for said effects hormonal therapies with estrogens are commonly used. Currently, most of the symptoms associated with menopause can be successfully prevented by means of hormonal replacement therapy. However this treatment by estrogen administration suffers of potential risks. Said risks include endometrial or breast tumor potentiation or thromboembolyc, hepatic or gallbladder alterations- Also, this type of therapy is counter-indicated in cases with antecedents of estrogen-dependent tumors in breast or endometrium.

Currently, an increasing interest has been generated in the use of phytoestrogens that could offer a potential health benefit provided by a diet rich in these compounds, and the positive effects on the hormone-dependant processes. Particularly, the main focus of attention of phytochemical products is centered on the isoflavones group.

Isoflavones are secondary vegetable substances, which are found mainly in species from the Fabaceae family, such as soy bean (*Glycine max*) or red clover (*Trifolium pratense*); and having biological properties affecting a diversity of biochemical and physiological process of human body. Some isoflavones, together with some lignanes, coumestanes and resorcinol derivatives, form part of phytoestrogens, are those compounds of non-steroidal nature that exhibit an estrogen agonist activity due to its interaction with 17-β-stradiol receptors. Isoflavones have two hydroxyl groups in an arrangement and at a distance similar to those of estradiol thereby interacting with estrogen α and β receptors. They are 3-receptor specific, whereby their effects occur in organs where said receptors prevail (CNS, bone, vascular wall, urogenital tract). Although the isoflavone effects are not potent, concentrations reached in the body are highly enough to elicit their physiological effects.

The physiological effects of isoflavones occur at various levels, therefore, for example, it is known that isoflavones have a great potential on hormonal effects since they regulate estrogen levels in the body helping to reduce menopausal effects (climacteric symptoms); also isoflavones exhibit anti-atherogenic activity, reduce the occurrence of osteoporosis, exhibit an hipolipidemic, antioxidant effect, isoflavones are also inhibitors of a plurality of enzymes, they exhibit vasodilating activity, anti-platelet aggregation activity, they protect from prostate disorders, and also exerts anti-tumoral and anticancer actions.

Referring to climacteric symptoms, due to a weak estrogenic effect of isoflavones, they can antagonize low estrogen levels symptoms associated to beta-receptors such as hot flushes, light sweats, and osteoporosis, while not affecting others symptoms such as uterine atrophy associated to alpha-receptors. It also appears that they don't have effects on depression, anxiety and insomnia associated with menopause. Some authors (Nagata C, Takasuka N, Kawakami N et al. "Soy product intake and hot flashes in Japanese women results from a community-based prospective study-" Am J Epidemiol 2001,153(8) 79)-3); carried out a prospective study in 1,106 pre-menopausal women with ages ranging between 35 and 54. They were given a soy protein-rich diet with isoflavone, during six years. Only 101 of said women reported suffering from menopausal hot flashes. It was concluded that the probability of suffering said hot flashes was inversely proportional to soy isoflavones consumption.

Other authors (Somewaka Y., et al. "Soy intake related to menopausal symptoms, serum lipids, and bone mineral density in postmenopausal Japanase women", Obstet Gynecol 2001,97(1) 109-15) evaluated the effects of soy in a prospective study on 478 postmenopausal women. Women were assigned to two groups according to years since menopause (early and late menopausal); and each group was subdivided into four groups according to the level of dietary isoflavone intake, with a mean of 54.3 mg/day- It was demonstrated that isoflavones significantly decreases palpitations, backaches, bone mass loss and hot flushes, specially in the early menopause group A clinical double-blind, randomized, placebo-controlled, parallel group, multicenter (in 5 sites) study (Upmalas DH, Lobo R, et al Vasomotor symptom relief by soy isoflavone extract tablets in postmenopausal women: a multicenter, double-blind, randomized, placebo-controlled study. Menopause 2000,7(4) 236-42) was carried out in 117 postmenopausal women with a mean age of 55 years, who experienced five or more hot flashes per day. They were administered 50 mg of isoflavone per day or placebo during 12 weeks. Isoflavones provided a significant reduction of hot flushes since week 2.

On the other hand, it has been demonstrated in animal and human studies that isoflavones increase bone density. The effect appears to be more an increase in bone building than a decrease in bone resorption. Isoflavones have shown to be effective only as preventatives, being unable to revert an established osteopenia.

Certain authors (Alekel DL, Germain AS, et al. Isoflavone-rich soy protein isolate attenuates bone loss in the lumbar spine of perimenopausal woman. Am J Clin Nutri 2000; 72(3) :844-52) carried out a 24 weeks ramdomized and double-blind study in 69 postmenopausal women. Three groups were established control group, isoflavone-poor soy group, isoflavone-rich soy group. It was observed that an 80.4 mg/day isoflavone dose attenuated bone loss of femur and lumbar spine of said women because it increases bone density in 5.6% and bone mineral content in 10.1%.

Other researchers contrasted the aforementioned data (Potter SM, et al Soy protein and isoflavones: their effects on blood lipids and bone density in postmenopausal women. Am J Clin Nutri 1998; 68(6 suppL) :1375S-1379S) in a 6 months, double-blind, randomized, controlled, parallel-group trial, in 66 postmenopausal and hypercholesterolemic women. They confirm that the administration of 40 g/day of soy proteins with 2.25 mg of isoflavones per gram of protein significantly, reduces spinal bone mass loss.

Furthermore, phytoestrogens have been shown to have a protective effect on the cardiovascular system, reducing the risk of arteriosclerosis in postmenopausal women. Although the exact mechanism of action it is unknown, it seems that this effect is lowed to two possible effects: the hypolipidemic effect, in which isoflavones provide an improvement on the lipid profile due to its estrogenic effect, and its action on the bile acids balance. Also, it seems that isoflavones increases LDL receptors. In animal studies, soy isoflavones provide an improvement on the HDL and LDL levels and a decrease on the risk of arteriosclerosis. Some authors (Goodman-Gruen D Usual Dietary isoflavone intake is associated with cardiovascular disease risk factors in postmenopausal women J Nutri 2001, 131 4) 1202-6) in a randonized, double-blind, cross-sectional and placebo-controlled clinical study in 208 postmenopausal women aged 45-75 years confirm that isoflavones decrease the body mass index and significantly increase HDL levels. And the antioxidant effect, where isoflavones exhibit, as all phenolic compounds do, an antioxidant effect. Therefore isoflavones protect LDL from oxidation and prevent the occurrence of foamy cells. In assays *in vitro,* it has been observed that isoflavones bind to LDLs and inhibit copper-mediated oxidation thereof.

Isoflavones are inhibitors of a multitude of enzymes, among which tyrosine-kinase is the most prominent, the inhibition of this enzyme triggers two major processes, namely the inhibition of expansion of certain oncogenes and the decrease of receptors for some cell proliferation factors (epidermal growth factor, insulin type growth factor, platelet growth factor, tumoral growth factor. Topoisomerase It wherein isoflavones form a ternary complex with the DNA-topoisomerase. It complex inducing cancer cell apoptosis. Aromatase is another enzyme that could be inhibited by isoflavones, wherein isoflavones prevented 17-β-estradiol formation from testosterone, acting on hormone dependant tumors such as breast cancer.

Isoflavones have demonstrated on in vitro and in vivo trials that they are able to prevent the α-TNF-induced activation of kappa-B transcription factor. Certain authors (Nakagawa H, et al. Effects of genistein and synergistic actino in combination with eicosa-pentaenoic acid on the growth of breast cancer cell lines. J. Cancer Res Clin Oncol 2000; 126(8):44 -54) found in trials in vitro in carcinogenic breast cells that genistein in a dose of 185 µM exhibit a dose- and time-dependant inhibitory effect of said cell lines.

Regarding prostate carcinoma, it has been demonstrated in animal and humans trials that isoflavones exhibit a preventive effect. It was demonstrated, in vitro, (Hillman GG, et al. Genistein potentiates the radiation effect on prostate carcinoma cells Clin Cancer Res 2001; 7 (2) : 382-90 that genistein, at 15 µM dose, exhibit a potentiating effect of radiotherapy in the treatment of prostate carcinoma, due to affectation of cell cycle and the production of apoptosis.

Regarding human, breast carcinoma, the effects of isoflavones on the growth of cell lines were examined. It was found that genistein is a potent inhibitor of every cell line [IC₅₀ value (concentration for 50% inhibition) of 6.5 to 12 0 micrograms/ml] (Peterson G and Barnes S (1991) Genistein inhibition of the growth of human breast cancer cells: independence from estrogen receptors and the multidrug resistant gene. Biochem Biohys Res Commun 179(1) 2868-74). Isoflavone genistein attenuate:; proliferation of growth factor and the stimulation of citokines from normal and cancerous cells (Am J Clin Nutri 1998, 68 (suppl) 1418S-25S).

Isoflavones have other important actions in the organism; for example, isoflavones reduce the levels of free calcium inside smooth muscle cell by reducing calcium influx from the exterior and increasing calcium reuptake by sarcoplasmic reticulum. They also stimulate nitric oxide synthase (NO). These effects together are synergized to each other to achieve the vasodilator effect. This vasodilator action has been demonstrated in animal trials. Another example is that isoflavones inhibit tromboxane A2 and tyrosine kinase. In assays in vitro on platelets (Nakashima S et al. Genistein, a protein tyrosine inhibitor, inhibits thromboxane A2-mediated human platelet responses Mol Pharmacol 1991, 39(4) 474-80) it was demonstrated that isoflavones competitively suppressed platelet aggregation in a dose-dependant fashion.

Most of researchers believe that isoflavones have excellent bioavailability when orally administered; likewise, the majority of investigations related to the role of isoflavones in estrogens physiology and physiopathology, is focused on genistein.

Isoflavones have' other important biological activities that could explain some of the beneficial effects such as prevention of several cancers and chronic diseases.

Isoflavones act as antioxidants to diminish pathophysiological damage associated with free radicals generation and oxidative stress. This oxidative activity can explain the effects of neurodegenerative diseases, which are believed result particularly from excess reactive oxygen, and nitrogen species produced by normal metabolic processes.

Isoflavones are also capable of inhibiting enzymes and receptors (epidermal growth factor and receptors for tyrosine kinase and topoisomerasa) that might play a role in the pathological damage seen in cancers and degenerative diseases.

The mechanism that partially explains the anticancer activity of isoflavones involves their ability to inhibit angiogenesis. Angiogenesis or growth of new blood vessels is required for tumor growth. Genisteine is able to block this process. Currently, one of the treatments for cancer involves angiogenesis inhibitors, wherein endostein is a synthetic agent that seems to have effect and genisteine is their natural counterpart.

On the other hand, the importance of others substances that help to prevent cancers, and in the prevention of other diseases and physiological imbalances, it is known, this way, for example, calcium sacarate is a calcium salt of D-glucaric acid contained in fruits and vegetalales. Calcium D-glucarate has been shown to inhibit beta-glucuronidase, an enzyme found in certain bacteria that reside in the gut. An elevated beta-glucuronidase activity has been associated to an increased risk of various cancers, especially hormone-dependent cancers such as breast, prostate and colon cancer. (Walaszek Z. Szemraj et al Metabolism, uptake, and excretion of a D-glucaric acid salt and its potential use in cancer prevention Cancer Detect Prev 1997 21 178-90).

Similarly, it is already known that the administration antioxidant vitamins, improves nutrient levels in blood, even in women with a relatively good diet.

Oxidative stress is associated to ageing and to the risk of developing certain diseases, to palliate this effect, some authors propose the benefits of incorporating antioxidants to the diet.

Data from the Health and Nutrition School in a national survey in USA were analyzed in order to detect the presence of food supplements in relaticn to life style and demographical characteristics. 52% percent of adults reported taking dietary supplements,in the past month; 35% reported having taken a multivitamin, calcium, vitamin E, B-complex, more than 5% reported having taking antacids.

In bivariate analyses, female gender, older age, more education, non-Hispanic White race/ethnicity, any physical activity, normal/underweight, the relationship with supplement and multivitamin corsumption was found Radimer K. Biendewald, Hughes J). In multivariable comparisons, the latter three characteristics were not associated with supplement use. Forty-seven percent of .the sample reported Having taken daily and for at least 2 years more than one supplement, also women aged >60 years took more than one supplement.

The protective effect of vitamins was demonstrated when vitamins were simultaneously applied. The level of DNA damage when vitamins were applied was lower then when they were not. The study was carried out according to several protocols of vitamins treatment nd the results shred evidence of the antitoxic activity of vitamins C and E. The activity of vitamins doesn't seem to have a connect on with the steps of DNA metabolic activation. This shows that the two vitamins act as competitors of DNA molecule in reaction to oxygen reactive species.

Vitamin A (retinol palmitate) plays diverse functions in the organism, acting as a cofactor for various biochemical reactions such as: cholesterol synthesis; mucopolysaccharides synthesis and hydroxylation of certain drugs. It plays main role in retina functioning and its essential for the integrity of skin and respiratory tract epithelial cells.

It is rapidly absorbed in the gastrointestinal tract, but its absorption can de decreased in the presence of poor absorption of fats, low intake of proteins, hepatic damage or pancreatic malfunctioning (Sodeman, Physiopathology, 7th edition).

Ascorbic acid (Vitamin C) acts like a reducing and antioxidant agent; it is essential for synthesis of collagen and intracellular material. It is rapidly absorbed in the gastrointestinal tract and is broadly distributed in all tissues; when there is a deficiency, blood white cells Concentration decreases (Sodeman, Physiopathology, 7th edition).

Tocopherol (Vitamin E) acts as antioxidant, in this action inhibits essential cell components oxidation and prevents the formation of toxic products resulting from oxidation. It is absorbed in the digestive tract by a mechanism similar to that of liposcluble vitamins and penetrates to bloodstream through lymph. It is stored in the tissues an these stores can protect against a deficiency during extended periods (Harrison, Principles of Internal Medicine, 13th edition, 558).

From the above, the great importance of isoflavones for the treatment of multiple physiological disorders it is evident, some of which are linked to the symptoms and disorders caused by menopause. Isoflavones intake can be performed together with direct intake or soy or soymilk.

In light of the importance of isoflavones positive effects in reducing or eliminating multiple physiological symptoms and disorders, it became necessary the availability of composition comprising isoflavones which in combination with other substances having positive physiological effects will generate a synergic effect that contributes to the elimination of physiological disorders caused by menopause.

### Object of the invention

The main object of the present invention is to make available an isoflavones composition for the treatment of physiological disorders and symptoms caused by menopause and climacterium as well as other emotional disturbances provoked by hormonal changes which characterized mature women.

### DETAILED DESCRIPTION OF THE INVENTION

Because of isoflavones, which modulate estrogenic activity, the investigations have been focused on the stages of an exclusive, or more prevalent disease in women: menopause.

After several studies it has been demonstrated that the intake of isoflavones plays a critical role in reducing the risk of acquiring diseases. Intestinal flora bacteria transform isoflavones during digestion, once transformed by the intestine, they exert their beneficial effects on the body.

Estrogens, like all hormones, act by using receptors located on the cell, which provokes some reaction. The alpha-receptors are linked with a risk of cancers; on the other hand, the beta-receptors are associated only to favorable effects. The repartition of these two types of receptors in the cells and organs is different; different fine tissues appear to have different ratios of each receptor type

This discovery allowed us to understand why isoflavones can act differently than estrogens, even though the chemical structure of isoflavones is similar to that of estrogens. Isoflavones exhibit a weak estrogenic action, 1/1000 to 1/100,000 of estradiol activity. When the natural levels of estrogens are low, isoflavones can help the estrogens by activating the beta-roceptors. When the natural levels of estrogen are high, for example during adolescence, the isoflavones bind with the alpha-receptors and prevent the natural estrogens from binding with these receptors.

Isoflavones activate the beta-receptors and reinforce the favorable estrogenic properties. On the other hand, isoflavones protect the estrogen alpha-receptors; consequently the proneness to estroger-related cancers is lower. The beta-receptors, which exercise favorable effects for health, can be found mainly in blood cells, lungs, prostate, bladder, bones and thymus. Isoflavones stimulate their function even after the level of estrogens has decreased. The alpha-receptor can be found mainly in breast tissue, uterus, ovaries, testicles and liver; in those places, isoflavones protect the receptcr from estrogens and help reduce the proneness to tumors.

After ingestion, isoflavones are hydrolyzed by intestinal glucosidases in the intestire, which release the aglycones (daidzein, genistein and glycitein), they may be absorbed or metabolized to many specific metabolites. The more beneficial soflavones are genistein (formula I) and daidzein (formula II); genistein is the most potent antioxidant among soy isoflavones, followed by daidzein.

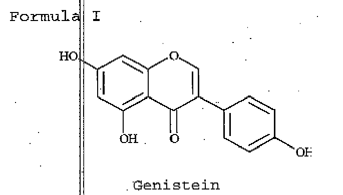

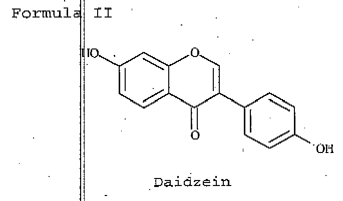

The half-life of daidzein and genistein was determined, measured in plasma with appearance and disappearance curves, which are of 7 9 hours in adults; peak concentrations are reached in 6-8 hours after administration. Plasma concentrations of 50-800 ng/ml of daidzein and genistein are reached, with doses of 50 mg/day of isoflavones. Excretion of isoflavones in urine take place within 24 hours and no more of 30% of ingested isoflavones dose can be quantified by urine concentrations and human plasma and excretion in feces is too low.

Isoflavones used in the present composition are 40% soy-isoflavones obtained from soybean powder which was subject to extraction employing alcohol at a concentration of ¼ of volume; the concentrated alcohol extract undergoes hydrolysis in order to obtain an concentrate in the form of paste (concentrated aqueous extract. This paste was crystallized from alcohol to obtain soy-isoflavones which are mixed with maltodextrine to obtain finally 40% soy-isoflavones.

Isoflavones composition for the treatment of menopausal physiological symptoms and disorders according to the present invention comprises a liquid solid or semisolid composition that includes *Glycine* max isoflavones, where the components are daidzein and genistein, at least one or a mixture of vitamins selected from vitamin A, Niacin, vitamin B, vitamin C, vitamin D, Folic acid and/or vitamin K, pantothenic acid, and at least one or a mixture of minerals selected from chrome, magnesium, manganese, calcium, copper, zinc, iron, potassium, phosphor or any other for human consumption.

Preferably the composition includes isoflavones, calcium sacarate vitamin C, vitamin E and vitamin A.

The composition provides a pharmaceutical formula containing per dosage unit: a) from 5 to 45% isoflavones, preferably ranging from 30 to 40%, for example 35.6%; b) from 5 to 55% calcium sacarate, preferably ranging from 30 to 40%, for example 35.6%; c) from 5 to 20% vitamin C, preferably in ranges from 7.5% to 15%, for example 10.67%; d) from 2.5 to 30% vitamin E, preferably in ranges from 10 to 20%, for example 17.8% and e) from C.15 to 3% vitamin A, preferably in ranges from 0.3 to 0.4%, for example 0.36%.

The above was demonstrated by performing studies of the combination of isoflavones, calcium sacarate and vitamins A, C and E, which showed a synergistic effect, compared to the administration of isoflavones alone.

The studies involved 200 female subjects, aged 40 to 45 years; all of the subjects meet the selection criteria, which were diagnosed as menopausal women exhibiting characteristics symptoms such as, perspiration, hot flushes, irritability, and depression.

Once they fulfill the criteria and signed the informed consent form to participate in the study, the subjects were divided into two groups, both groups were asked to answer questionnaire about the severity of their symptoms, to obtain initial data, subsequently group I received isoflavones alone once a day during a year and group II received the combination once a day during a year.

Consecutive visits were made every three months, using the questionnaire to evaluate the severity of symptoms and observe any adverse effect that might occur during the study.

### Results

All of the 200 female subjects concluded the study and they did not report any adverse event during its administration.

| Basal questionnaire | | | |
|---|---|---|---|
| Symptoms | Group I | Group II | p value |
| Perspiration | 100% | 100% | 0.05 |
| Hot flashes | 98% | 96% | 0.05 |
| Irritability | 85% | 80% | 0.05 |
| Depression | 95% | 95% | 0.05 |

There is not a statistical significance

| 1st visit questionnaire (3 months) | | | |
|---|---|---|---|
| Symptoms | Group I | Group XI | p value |
| Perspiration | 90% | 80% | 0.04 |
| Hot flashes | 85% | 80% | 0.05 |
| Irritability | 80% | 70% | 0.04 |
| Depression | 70% | 60% | 0.04 |

| | | | |
|---|---|---|---|
| p value = < 0.05 statistical significance | | | |

| 2nd visit questionnaire (6 months) | | | |
|---|---|---|---|
| Symptoms | Group I | Group II | p value |
| Perspiration | 85% | 70% | 0.04 |
| Hot flashes | 80% | 70% | 0.04 |
| Irritability | 70% | 60% | 0.04 |
| Depression | 60% | 50% | 0.04 |

| | | | |
|---|---|---|---|
| p value = < 0.05 statistical significance | | | |

| 3rd visit questionnaire (9 months) | | | |
|---|---|---|---|
| Symptoms | Group I | Group II | p value |
| Perspiration | 80% | 65% | 0.04 |
| Hot flashes | 80% | 50% | 0.03 |
| Irritability | 65% | 40% | 0.03 |
| Depression | 60% | 40% | 0.03 |

| 4th visit questionnaire (12 months) | | | |
|---|---|---|---|
| Symptoms | Group I | Group ::I | p value |
| Perspiration | 70% | 35% | 0.02 |
| Hot flashes | 70% | 35% | 0.02 |
| Irritability | 55% | 20% | 0.04 |
| Depression | 55% | 20% | 0.02 |

| | | | |
|---|---|---|---|
| p value = < 0.05 statistical significance p value = << 0.05 statistical significance | | | |

### Discussion and conclusions

The improvement regarding symptoms during the study was evident in the group of patients treated with the combination compared to the group that received isoflavones alone, although it was demonstrated that said improvement is progressive during the course of the months, the statistical difference was very significant in both groups.

It is known the role of iscflavones because of their antioxidant effects, however vitamins' activity on isoflavones show a synergistic activity, we know about the effect of vitamin A as cofactor in several biochemical reactions such as cholesterol synthesis; of vitamin C that plays a role as reducing agent and antioxidant, essential for the synthesis of collagen and intracellular material; of vitamin E which acts as antioxidant by inhibiting the oxidation of essential cell components and prevents the formation of toxic products from oxidation.

The research team reached to the conclusion of the higher effectiveness of a combination of antioxidant substances like that utilized in this study compared to the single administration of isoflavones having antioxidant activity.

The invention has been described sufficiently such that one of ordinary skill in the art could reproduce and obtain the results mentioned in the present invention However, any one of ordinary skill in the art to which this invention pertains could be able to make modifications that were not described in the present application, however if the subject matter claimed in the following claims is required for the application of said modifications into a given structure or in the manufacturing process thereof, said structures are within the scope of the invention.

## Claims

1. An isoflavones composition for the treatment of menopausal physiological disorders and symptoms, wherein it comprises *Glycine max* isoflavones, at least one or a mixture of vitamins and at least one or a mixture of minerals.

2. The composition of isoflavones for the treatment of menopausal physiological disorders and symptoms of claim 1, wherein said at least one or a mixture of vitamins of said composition is selected from the group consisting of Vitamin A, Niacin, Vitamin B, Vitamin C, Vitamin D, Vitamin E, folic acid and/or Vitamin K, pantothenic acid

3. The composition of isoflavones for the treatment of menopausal physiological disorders and symptoms of claim 1, wherein said at least one or a mixture of minerals is selected from the group consisting of Chrome, Magnesium, Manganese, Calcium, Copper, Zinc, Iron, Potassium, Phosphor or any other for human consumption.

4. The composition of isoflavones for the treatment of menopausal physiological disorders and symptoms according to claims 1 to 3, wherein said composition preferably includes isoflavones, calcium Sacarate, Vitamin C, Vitamin E and Vitamin A.

5. The composition of isoflavones for the treatment of menopausal physiological disorders and symptoms according to claim 4, wherein said composition provides a pharmaceutical composition containing per dosage unit: a) from 5 to 45% isoflavones, preferably ranging from 30 to 40%; b) from 5 to 55% calcium sacarate, preferably ranging from 30 to 40%; c) from 5 to 20% vitamin C, preferably in ranges from 7.5% to 15%; d) from 2.5 to 30% vitamin -E, preferably in ranges from 10 to 20%% and e) from 0.15 to 3% vitamin A, preferably in ranges from 0.3 to 0.4%%.

6. The composition of isoflavones for the treatment of menopausal physiological disorders and symptoms according to any one of claims 1 to 5, wherein the composition have a liquid, solid or semisolid presentation.
